Europäisches Patentamt

European Patent Office

Office européen des brevets

(11) Publication number: **0 251 746 B1**

# EUROPEAN PATENT SPECIFICATION

(45) Date of publication of patent specification: **27.11.91**   (51) Int. Cl.⁵: **A61N 1/06, A61N 1/40**

(21) Application number: **87305734.3**

(22) Date of filing: **26.06.87**

(54) **A dielectric-heating electrode device for hyperthermia.**

(30) Priority: **27.06.86 JP 98429/86 U**
**27.06.86 JP 98430/86 U**

(43) Date of publication of application:
**07.01.88 Bulletin 88/01**

(45) Publication of the grant of the patent:
**27.11.91 Bulletin 91/48**

(84) Designated Contracting States:
**DE FR GB IT**

(56) References cited:
**EP-A- 0 115 420       EP-A- 0 139 433**
**WO-A-80/01045         DE-A- 2 407 559**
**GB-A- 514 466         US-A- 2 882 904**

**SOVIET INVENTIONS ILLUSTRATED, section
P/Q, week 8502, P34, abstract no.
85-010720/02, 20th February 1985, Derwent
Publications Ltd, London, GB; & SU-A-1 093
347 (TSELINOGRAD MED. INS.) 23-05-1984**

(73) Proprietor: **OLYMPUS OPTICAL CO., LTD.**
**43-2, 2-chome, Hatagaya Shibuya-ku
Tokyo 151(JP)**

(72) Inventor: **Sogawa, Akira
105 Guroria-Haitsu 3-17-5 Toyotama-Minami
Nerima-ku Tokyo(JP)**
Inventor: **Kitagawa, Kiyoshi 2-503 Makuhari-
Famiiru-Haitsu
5-417-16 Makuhari-cho Chiba-shi
Chiba-ken(JP)**
Inventor: **Onodera, Chikau
4-25-27 Kasuga-cho Nerima-ku
Tokyo(JP)**
Inventor: **Onuma, Tadashi
2213-4 Takasaki Kukizaki-machi
Inashiki-gun Ibaraki-ken(JP)**

(74) Representative: **Senior, Alan Murray et al
J.A. KEMP & CO 14 South Square Gray's Inn
London WC1R 5EU(GB)**

## Description

This invention concerns a heating electrode device for medical use and, particularly, it relates to a high-frequency heating electrode device for medical use that can be applied to the hyperthermia of tumours, etc.

High-frequency hyperthermia has been known in which therapy is carried out by applying heat to a lesional portion of a patient, utilizing the fact that cancer cells, etc are less resistant to heat than normal cells.

In the conventional high-frequency heating method, hyperthermia is carried out by putting a region including an aimed portion to be heated of a living body between two opposed plate-like electrodes and applying a high-frequency current between the electrodes from a high-frequency generator.

In this case, because of the difference in electrical constants (electroconductivity and dielectric constant) between a subcutaneous fat layer and an endotract organ tissue, the subcutaneous fat layer tends to be heated more intensely, or a patient may complain of undesirable feeling of heat, or the epidermatic tissue may suffer from the danger of burn due to the intense heating at a portion near the electrode body. Accordingly, there has been proposed a method of attaching a flexible and gastight bag member between a living body and electrode body and filling or circulating a coolant into or through the inside of the bag member. EP-A-0115420 discloses an electrode device having a bag-like member through the inside of which a coolant is circulated, and EP-A-0139433 discloses an electrode device for use on an outer surface of a living body, and having a bag-like member through the interior of which a coolant is circulated. However, although there is no trouble so long as the device operates normally, if any unexpected accident should occur such as change of the introduction pressure, interruption or leakage of a coolant, the electrode body may possibly be brought into a close contact with the living body thereby causing an anxiety about safety.

The present invention has been achieved in view of the foregoing problems and, accordingly, it is an aim thereof to provide a high-frequency heating device capable of deforming in accordance with the shape of a body surface (inner surface of a tubular endotract organ), capable of surely and safely heating a predetermined region near the body surface selectively and keeping the electrode body from being brought into directly close contact with the body surface.

According to the present invention there is provided an electrode device for heating a tumor in an endotract organ by high frequency current, wherein said electrode device is deformable and includes:

a relatively thick wall made of a flexible polymeric material;

a flexible polymeric film attached to said thick wall so as to define a closed space between said thick wall and said flexible polymeric film;

an easily deformable electrode member disposed on an inner surface of said thick wall;

a cable electrically connected to one end thereof to said electrode member so as to supply a high frequency current to said electrode member;

and means for supplying and discharging a coolant to and from said closed space, characterised in that:

said electrode device is insertable into said endotract organ,

said thick wall forms a container comprising a thick side wall pierced with said cable, a thick bottom wall connected to one end of said side wall, and an opening defined by the other end of said side wall, said flexible polymeric film is fluid-tightly attached to said side wall to close said opening and

said electrode device further comprises a mesh-like member made of a flexible polymeric material, and disposed on a surface of said electrode member, which faces said flexible polymeric film, to prevent close contact between said flexible polymeric film and said electrode member.

According to an optional feature of the present invention, an electrode device of the present invention can be so adapted that the electrode device can be inserted into a tubular endotract organ (for example, vagina), and that the electrode device can be secured to the portion which is intended to be heated on the inner surface of the tubular endotract organ without separately preparing the device to secure it in the predetermined position.

These and other objects, as well as other advantageous features of the present invention will now be described more specifically while referring to the accompanying drawings, wherein

Figures 1 and 2 are explanatory views for high-frequency hyperthermia;

Figure 3 is an explanatory view illustrating one embodiment of the electrode device according to the present invention;

Figure 4 is a cross-sectional view showing the electrode device of Figure 3; and

Figure 5 is an explanatory view illustrating a further embodiment of the present invention.

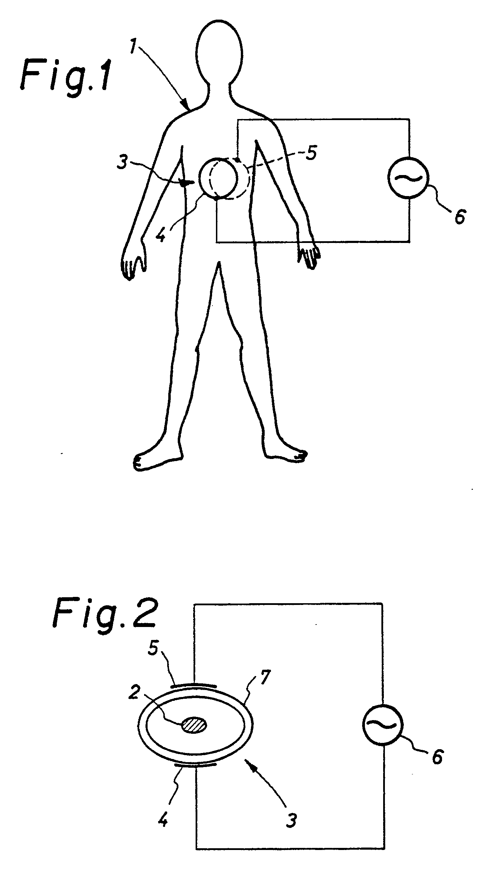

In the conventional high-frequency heating method, hyperthermia is carried out as shown in Figures 1 and 2, by putting a region 3 including an aimed portion 2 to be heated of a living body 1 between two opposed plate-like electrodes 4 and 5 and applying a high-frequency current between the electrodes 4 and 5 from a high-frequency generator 6.

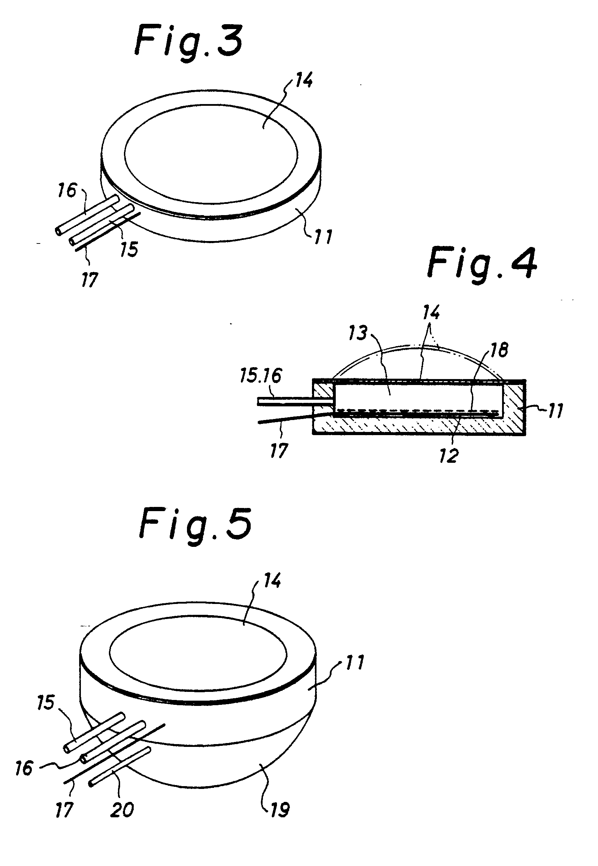

In Figures 3 and 4, a thick walled container 11 made of a flexible polymeric material, for example, synthetic or natural rubber of excellent adaptability with the shape of a living body to be applied, for example, silicone rubber. The shape and the size of the container 11 may optionally be designed in accordance with the purpose of use. For example, in a case of an electrode device for vagina, it may be a flat elliptic shape having outer diameter of 30 - 90 mm, wall thickness of 8 - 25 mm and depth of 5 - 15 mm in view of easy installation and withdrawal. An electrode member 12 is disposed to the inner surface of the container 11 on the side opposite to the open surface of the container. The electrode member 12 is made of highly electroconductive material such as copper and silver and can be formed, for example, in an easily deformable plate-like, foil-like or mesh-like shape. The shape of the electrode member is not necessarily dependent upon the outer shape of the container 11. The electrode member 12 is provided with a conductor 17 which passes through the wall of the container and is connected with a high-frequency generator (not illustrated). The open surface of the container 11 is closed by a flexible polymeric film 14 so as to form a gastight space 13 within the container. The space 13 is so adapted that a coolant such as water or brine can be filled into or circulated through supplying and discharging channels 15 and 16 disposed to the container 11 from coolant charging and discharging means (not illustrated). The coolant is used for the purpose of preventing the localized heating, as well as for firm contact of the electrode with the aimed portion upon hyperthermia. For effectively attaining the purpose of using the coolant, the flexible polymeric film 14 can include, for example, an extensible or expandable polymeric film made of natural or synthetic rubber with the film thickness of about 0.2 - 0.5 mm. Further, a sensor for monitoring the heated state may previously be disposed to the outer surface of the polymeric film 14 if required.

As seen in Figure 4, a mesh-like member 18 made of polytetrafluoroethylene is disposed to the surface of the electrode member so as to prevent close contact between the flexible polymeric film 14 and the electrode member 12.

Figure 5 shows a further embodiment of the present invention in which a gas-tight bag member is attached to the rear face of the electrode device shown in Figures 3 and 4. Parts identical with those in Figures 3 and 4 carry the same reference numerals. The gastight bag member 19 is bonded by means of adhesives or the like to the rear face of a thick walled container 11 and adapted to supply or discharge air or like other gas through a conduit 20. The gastight bag member 19 may be formed, for example, by shaping a flexible film or tube such as made of polyethylene or polypropylene into a predetermined configuration. However, a molding member or balloon made of silicone rubber or natural rubber that can easily expandable or extensible with an introduced gas is preferred in that the electrode device inserted into a tubular endotract organ, for example, to the inside of a vagina can surely be secured and retained.

Although a one-path structure is illustrated for the channel for flowing gas to the inside of the gastight bag member 19 in Figure 5, a plurality of gas flowing channels may be formed.

The electrode devices according to the present invention shown in Figures 3 through 5 are used for high-frequency hyperthermia in combination with a counter electrode device in actual use. In this case, the temperature on the side where the electrode device according to the present invention is used can be set to a temperature optimum to the therapy by using the counter electrode device which comprises an electrode member (non-sensitive electrode) having an electrode area greater than ten times as that of the electrode member of the electrode device according to the present invention. Further, in the case of actually using the electrode device shown in Figure 5, the electrode device is inserted into the inside of the tract of a living body in a state where the gastight bag member is deflated, and situated such that the open surface of the thick walled container is directly faced with the aimed portion to be heated. Then, the coolant is introduced through the supplying and discharging channels 15 and 16 to the space 13. Further, air or like other gas is introduced through the conduit 20 to the gastight bag member 19 so that the electrode device is secured to the aimed portion to be heated at an appropriate pressure by utilizing the expansion of the gastight bag member 19. Since the heating electrode is in conformity with and in close contact with the body surface and the electrode member is kept from a close contact with the living body even in a case where coolant is not present in the space within the thick walled container due to the structure in which the electrode member is disposed within the thick walled container, the hyperthermia can be conducted safely.

Further, the electrode device which has the gastight bag member can provide a merit that the electrode device can be secured to the aimed portion to be heated and the intrusion of the high-frequency current to the side of the gastight bag member of the thick walled container which is expanded with the gas can be decreased as less as possible.

## Claims

1. An electrode device for heating a tumor in an endotract organ by high frequency current, wherein said electrode device is deformable and includes:
   a relatively thick wall made of a flexible polymeric material;
   a flexible polymeric film (14) attached to said thick wall so as to define a closed space between said thick wall and said flexible polymeric film;
   an easily deformable electrode member (12) disposed on an inner surface of said thick wall;
   a cable (17) electrically connected at one end thereof to said electrode member so as to supply a high frequency current to said electrode member;
   and means for supplying and discharging a coolant (15, 16) to and from said closed space, characterised in that:
   said electrode device is insertable into said endotract organ,
   said thick wall forms a container (11) comprising a thick side wall pierced with said cable (17), a thick bottom wall connected to one end of said side wall, and an opening defined by the other end of said side wall, said flexible polymeric film (14) is fluid-tightly attached to said side wall to close said opening and
   said electrode device further comprises a mesh-like member (18) made of a flexible polymeric material, and disposed on a surface of said electrode member (12), which faces said flexible polymeric film (14), to prevent close contact between said flexible polymeric film (14) and said electrode member (12).

2. An electrode device according to claim 1, wherein said container (11), made of said flexible polymeric material, has a flat elliptic shape.

3. An electrode device according to claim 1 or 2, wherein said electrode member (12) has a plate-like shape and is foil-like or mesh-like.

4. An electrode device according to any one of claims 1 to 3, wherein said flexible polymeric film (14) is an extensible or expandable film made of natural or synthetic rubber.

5. An electrode device according to any one of claims 1 to 4, wherein said flexible polymeric material for said mesh-like member (18) is polytetrafluoroethylene.

6. An electrode device according to any one of claims 1 to 5, further comprising: a flexible and gastight bag member (19) made of an expandable material, and disposed on an outer surface of said thick bottom wall which is opposite to said flexible polymeric film (14), to thereby urge said electrode device against an aimed portion to be heated of said endotract organ when said electrode device is inserted into said endotract organ and said flexible and gastight bag member (19) is inflated; and means (20) connected to said flexible and gastight bag member for supplying and discharging a gas into and out of a space defined by said outer surface of said thick bottom wall and said flexible and gastight bag member.

7. An electrode device according to claim 6, wherein said gastight bag member (19) comprises a molding member or balloon made of synthetic or natural rubber which is easily extensible or expandable by said introduced gas.

## Revendications

1. Dispositif à électrode pour chauffer une tumeur dans un organe endotractile par un courant à haute fréquence, dans lequel ledit dispositif à électrode est déformable et comporte :
   une paroi relativement épaisse en un matériau polymère flexible ;
   un film polymère flexible (14) fixé à ladite paroi épaisse, de manière à définir un espace fermé entre ladite paroi épaisse et ledit film en polymère flexible ;
   une électrode facilement déformable (12) disposée sur une surface intérieure de ladite paroi épaisse ;
   un câble (17) connecté électriquement à une extrémité de ladite électrode de manière à alimenter en courant à haute fréquence ladite électrode ;
   et des moyens (15, 16) pour alimenter et décharger en agent de refroidissement ledit espace fermé, caractérisé en ce que :
   ledit dispositif à électrode peut être inséré à l'intérieur dudit organe endotractile,
   ladite paroi épaisse formant un réceptacle (11) comporte une paroi latérale épaisse percée par ledit câble (17), un fond à paroi épaisse connecté à une extrémité de ladite paroi latérale et une ouverture définie par l'autre extrémité de ladite paroi latérale, ledit film polymère flexible (14) étant fixé de façon étanche au fluide, à ladite paroi latérale pour fermer ladite ouverture et, en ce que,
   ledit dispositif à électrode comporte en outre un élément en forme de grille (18) constitué par un matériau polymère flexible et disposé sur une surface de ladite électrode (12) qui fait face audit film polymère flexible (14) pour empêcher un contact étroit entre ledit film polymère flexible (14) et ladite électrode (12).

2. Dispositif à électrode selon la revendication 1, dans lequel ledit réceptacle (11), constitué par ledit matériau polymère flexible, a une forme elliptique plate.

3. Dispositif à électrodes selon les revendications 1 ou 2, dans lequel ladite électrode (12) a une configuration de plaque et est sous forme de feuille ou de grille.

4. Dispositif à électrode selon l'une quelconque des revendications 1 à 3, dans lequel ledit film polymère flexible (14) est un film extensible ou dilatable en caoutchouc naturel ou synthétique.

5. Dispositif à électrode selon l'une quelconque des revendications 1 à 4, dans lequel ledit matériau polymère flexible pour ledit élément en forme de grille (18) est du polytétrafluoroéthylène.

6. Dispositif à électrode selon l'une quelconque des revendications 1 à 5, et qui comporte en outre : un élément faisant sac étanche et flexible (19) en un matériau dilatable, disposé sur une surface extérieure dudit fond à paroi épaisse opposé audit film polymère flexible (14) afin de pousser ledit dispositif à électrode contre une partie ciblée devant être chauffée dudit organe endotractile, lorsque ledit dispositif à électrodes est inséré à l'intérieur dudit organe endotractile, ledit élément faisant sac étanche et flexible (19) étant gonflé ; et un moyen (20) connecté audit élément faisant sac étanche et flexible pour alimenter et décharger en gaz l'espace défini par ladite surface extérieure dudit fond à paroi épaisse et ledit élément faisant sac étanche et flexible.

7. Dispositif à électrode selon la revendication 6, dans lequel ledit élément faisant sac étanche (19) comprend un élément moulé ou ballon en caoutchouc synthétique ou naturel qui est facilement extensible ou dilatable par ledit gaz qui y est introduit.

**Patentansprüche**

1. Elektrodenanordnung zur Erwärmung eines Tumors in einem inneren Organ durch Hochfrequenzstrom, bei dem die Elektrodenanordnung deformierbar ist und enthält:

eine verhältnismäßig dicke Wand, die aus einem flexiblen polymeren Material gemacht ist; einen flexiblen polymeren Film (14), der an der dicken Wand befestigt ist, um so einen geschlossenen Raum zwischen der dicken Wand und dem flexiblen polymeren Film zu begrenzen; ein leicht deformierbares Elektrodenteil (12), das an der Innenfläche der dicken Wand angeordnet ist; ein Kabel (17), das an einem Ende mit dem Elektrodenteil verbunden ist, um dem Elektrodenteil Hochfrequenzstrom zuzuführen; und Mittel zum Zuführen und Abführen eines Kühlmittels (15, 16) zu und aus dem geschlossenen Raum, dadurch gekennzeichnet, daß die Elektrodenanordnung in das innere Organ einführbar ist, daß die dicke Wand einen Behälter (11) bildet, mit einer dicken Seitenwand, die von dem Kabel (17) durchsetzt ist, einer dicken Bodenwand, die mit einem Ende der Seitenwand verbunden ist, und einer Öffnung, die durch das andere Ende der Seitenwand definiert ist, wobei der flexible polymere Film (14) flüssigkeitsundurchlässig an der Seitenwand befestigt ist, um die Öffnung zu schließen, und daß die Elektrodenanordnung ferner ein maschenähnliches Teil (18), das aus einem flexiblen polymeren Material hergestellt und auf der Oberfläche des Elektrodenteils (12) angeordnet ist, aufweist, das dem flexiblen polymeren Film (14) gegenüberliegt, um so einen engen Kontakt zwischen dem flexiblen polymeren Film (14) und dem Elektrodenteil (12) zu verhindern.

2. Eine Elektrodenanordnung nach Anspruch 1, bei der der Behälter (11), der aus dem flexiblen polymeren Material hergestellt ist, eine flache elliptische Form hat.

3. Elektrodenanordnung nach Anspruch 1 oder 2, bei der das Elektrodenteil (12) eine plattenähnliche Form hat und folien- oder maschenähnlich ist.

4. Elektrodenanordnung nach einem der Ansprüche 1 bis 3, bei der der flexible polymere Film ein dehnbar oder nachgiebiger Film ist, der aus natürlichem oder synthetischem Gummi hergestellt ist.

5. Elektrodenanordnung nach einem der Ansprüche 1 bis 4, bei der das flexible polymere Material für das maschenähnliche Teil (18) Polytetrafluorethylen ist.

6. Elektrodenanordnung nach einem der Ansprüche 1 bis 5, die des weiteren enthält:

   einen flexiblen, gasdichten Ballon (19), der aus einem dehnbaren Material hergestellt ist und an der Außenfläche der dicken Wand, die dem flexiblen polymeren Film (14) gegenüberliegt, angeordnet ist, um dadurch die Elektrodenanordnung gegen einen ausgewählten zu erhitzenden Abschnitt des inneren Organs zu drücken, wenn die Elektrodenanordnung in das innere Organ eingeführt ist und der flexible und gasdichte Ballon (19) aufgeblasen ist; und Mittel (20), die mit dem flexiblen und gasdichten Ballon verbunden sind zum Zuführen und Abführen eines Gases in und aus einem Raum, der durch die Außenfläche der dicken Bodenwand und den flexiblen und gasdichten Ballon definiert ist.

7. Elektrodenanordnung nach Anspruch 6, bei der der gasdichte Beutel (19) ein Formteil oder einen Ballon enthält, der aus synthetischem oder natürlichem Gummi hergestellt ist, der durch das eingeführte Gas dehnbar oder nachgiebig ist.

Fig.1

Fig.2

# Fig.3

# Fig.4

# Fig.5